(19) Europäisches Patentamt
European Patent Office
Office européen des brevets

(11) EP 1 532 977 A1

(12) EUROPEAN PATENT APPLICATION
published in accordance with Art. 158(3) EPC

(43) Date of publication:
25.05.2005 Bulletin 2005/21

(21) Application number: 03761830.3

(22) Date of filing: 27.06.2003

(51) Int Cl.[7]: A61K 31/137, A61K 31/5375, A61P 31/10

(86) International application number:
PCT/JP2003/008221

(87) International publication number:
WO 2004/002464 (08.01.2004 Gazette 2004/02)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 28.06.2002 JP 2002190041

(71) Applicant: Sato Pharmaceutical Co. Ltd.
Tokyo 107-0051 (JP)

(72) Inventors:
- ISHIDUKA, S.;
c/o R & D Center SATO PHARMA. CO LTD
Shinagawa-ku, Tokyo 140-0011 (JP)
- TADA, T.;
c/o R & D Center SATO PHARMA. CO LTD
Shinagawa-ku, Tokyo 140-0011 (JP)
- KATAYAMA, M.;
c/o R & D Center SATO PHARMA. CO LTD
Shinagawa-ku, Tokyo 140-0011 (JP)
- YANAGIHARA, S.;
c/o R & D Center SATO PHARMA. CO
Shinagawa-KU, Tokyo 140-0011 (JP)
- SHIMIZU, T.;
c/o R & D Center SATO PHARMA. CO LTD
Shinagawa-ku, Tokyo 140-0011 (JP)

(74) Representative:
Ziebig, Marlene, Dr. Dipl.-Chem. et al
Anwaltskanzlei
Gulde Hengelhaupt Ziebig & Schneider
Wallstrasse 58/59
10179 Berlin (DE)

# (54) ANTIFUNGAL AGENTS FOR EXTERNAL USE

(57) The present invention provides an antifungal agent for topical use and remedy for athlete's foot that comprises a new combination of medicines and has a strong antifungal activity against both genus Trichophyton and genus Candida.

The antifungal agent for topical use and remedy for athlete's foot contain amorolfine with butenafine or terbinafine.



Printed by Jouve, 75001 PARIS (FR)

## Description

Technical Field:

The present invention relates to a composition for antifungal agents effective against dermatomycosis. In particular, the present invention relates to an athlete's foot remedy, which has an enhanced antifungal effect against microorganisms of the genus Trichophyton and the genus Candida.

Background Art:

The main pathogens responsible for athlete's foot are fungi of the genus Trichophyton and the genus Candida. The main cause of Athlete's foot is Trichophyton and is intractable. Therefore, in the treatment of athlete's foot caused by Trichophyton, the medicine must be applied to the affected part for a long period of time. The symptoms of athlete's foot subside in winter, which does not mean that athlete's foot has been completely recovered, but the action of the fungi have become weak in winter. On the other hand, the cases of athlete's foot caused by Candida are smaller in number than the cases of athlete's foot caused by Trichophyton. The patients suffering from athlete's foot caused by Candida are relatively easily cured as compared with those caused by Trichophyton.

In the actual situation for the treatment of athlete's foot, there is a problem that it is difficult to distinguish the kinds of the fungi causing the symptoms of athlete's foot from each other. Namely, whether the fungus causing athlete's foot belongs to the genus Trichophyton or the genus Candida can be only determined by a medical specialist via microscope or culture methods. Thus, the determination of the pathogen causing the athlete's foot is troublesome and it takes a long time.

Under these circumstances, it is demanded to develop an antifungal agent having an excellent effect in treating athlete's foot and having the following two advantages: 1) the antifungal agent is further improved in effect on the fungi of the genus Trichophyton as the main cause for athlete's foot and which are intractable; and 2) the antifungal agent is effectively usable for the treatment without distinguishing the fungi even when the fungi causing athlete's foot belongs to the genus Candida.

For solving the above-described problems, there were developed antifungal agents having an antifungal effect improved by combining an azole antifungal agent which is effective on the fungi of both genus Trichophyton and genus Candida, together with various medicines. As the antifungal agents composed of a combination of the azole antifungal agent with another medicine, there have been disclosed, for example, combinations of the azole antifungal agent, with an arylmethylamine antifungal agent (Japanese Patent No. 2581707), with a nicomycin derivative (Japanese Patent No. 2713755), with lysozyme (Japanese Patent Kokai No. Hei 9-20680), with a quaternary ammonium salt (Japanese Patent Kokai No. Hei 9-110690), with a lactoferrin hydrolyzate or an antibacterial peptide derived therefrom (Japanese Patent Kokai No. Hei 9-165342) and with a given antifungal agent or disinfectant such as Ciclopyrox olamine or Tolnaftate (Japanese Patent Kokai No. Hei 9-110693). As a medicine composed of the combination of two kinds of azole antifungal agents, a combination of clotrimazole and pyrrolnitrin was disclosed (Japanese Patent Kokai No. 2000-186037).

However, all of these medicines contain an azole antifungal agent. There has never been reported an antifungal agent composed of a combination of an antifungal agent other than the azoles, such as a morpholine antifungal agent, with another medicine and having the antifungal effect on the fungi of both genus Trichophyton and genus Candida.

The development of antifungal agents is focused on the development of a new medicine at present. A medicine comprising a new combination of ordinary antifungal agents has not been as developed.

Although the antifungal agents composed of medicines having antifungal effects exhibit the summation of the effect of the respective medicines in many cases, the medicines also exhibit the antagonistic effects in some cases. Thus, it cannot be foreseen at present whether or not the new combination of the antifungal agents exhibits the potentiation. For obtaining the antifungal agents having the potentiation, it is required to test each combination of the antifungal agents to confirm the effect thereof. Thus, it is not easy to find a new combination of the medicines having the potentiation.

The object of the present invention is to provide an antifungal agent or a medicine for treating athlete's foot, which has a high antifungal activity particularly against the fungi of the genus Trichophyton and also effective on those of the genus Candida.

Disclosure of the Invention:

After intensive investigations on the effects of the combination of amorolfine, i.e. a morpholine antifungal agent, with various antifungal agents conducted for the purpose of further improving the effect of amorolfine effective

on the fungi of both genus Trichophyton and genus Candida, the inventors have found that, when amorolfine is combined with butenafine (a benzylamine antifungal agent) or terbinafine (an arylamine antifungal agent), the antifungal effect of amorolfine on the fungi of the genus Trichophyton can be remarkably improved and, in addition, the antifungal effect thereof on those of the genus Candida can be remarkably improved.

Amorolfine has a very strong antifungal effect against Trichophyton and it also has an antifungal effect on against Candida, though the latter effect is not as strong as the former effect. It is well known that, although butenafine has a very strong antifungal effect against Trichophyton, it is ineffective against Candida. Although terbinafine has a very strong antifungal effect on Trichophyton, its effect on Candida is weaker than that of amorolfine and, therefore, it had not been regarded as an important medicine against Candida. Thus, it is unexpected that the combination of amorolfine with butenafine or terbinafine exhibits a potentiation not only on Trichophyton but also on Candida. The inventors have completed the present invention on the basis of this finding. The present invention relates to an antifungal agent for topical use or an agent for treating athlete's foot, which contains amorolfine together with butenafine or terbinafine.

Brief Description of the Drawings:

Fig. 1 is a graph showing the rate of cultured positive loci obtained in the culture tests of tinea infection models of guinea pigs.

Fig. 2 is a graph showing the average intensity of infection in the culture tests of tinea infection models of guinea pigs.

Mode for Carrying out the Invention:

The concept of "amorolfine" which is a morpholine antifungal agent includes salts thereof in this specification. As the salts, hydrochlorides are particularly preferred. The structural formula of amorolfine hydrochloride ((±)-cis-2,6-dimethyl-4-[3-[4-(1,1-dimethylpropyl)phenyl]-2-methylpropyl]morpholine monohydrochloride ($C_{21}H_{35}NO \cdot HCl$), molecular weight: 353.97) is as follows:

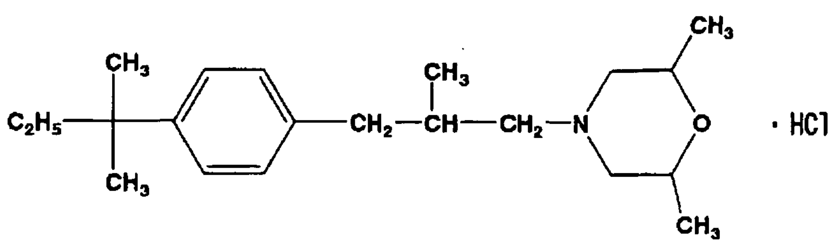

Amorolfine has broad antifungal spectrum against the genus Trichophyton and the genus Candida, as well as various other microorganisms of the genus Microsporum, Epidermophyton, Malassezia and dematiaceous fungi. Amorolfine is widely used in the world. In Japan, amorolfine is an antifungal agent most commonly used as a medicine at present, and it is easily available on the market.

The minimum inhibitory concentration (MIC) of amorolfine hydrochloride ranged from ≦ 0.0012 to 0.08 µg/mL against Trichophyton mentagrophytes, from ≦ 0.0012 to 0.02 µg/mL against Trichophyton rubrum, from 0.01 to 10 µg/mL against Candida albicans (as for MIC against clinical isolates from patients with cutaneous mycosis: refer to Hideyo Yamaguchi, et al., Jap. J. Antibiotics, 44(9), 1007, 1013 (1991)).

As for the mechanism of the function of amorolfine hydrochloride, it selectively inhibits two steps in the pathway of the ergosterol biosynthesis to disturb the structure and function of the cell membranes, thereby expressing the antifungal activity.

The toxicity of amorolfine hydrochloride is as follows:

$LD_{50}$ (mg/kg) mice: oral = male 2514, female 2406, subcutaneous > male and female 5000, intravenous injection = male 141, female 112, intraperitoneal injection = male 205, female 239;

Rats: oral = male 1960, female 1756, subcutaneous > male and female > 2000, intraperitoneal injection = male 468, female 465;

Dogs: oral > male and female 1000, subcutaneous > male= 2500;

Reproduction tests: (rats, oral): an influence of amorolfine hydrochloride on the survival rate of newborn babies was recognized after the administration thereof in a dose of at least 10 mg/kg/day in a period ranging from before the pregnancy to the lactation period or perinatal period/lactation period (see *Iryo-yaku Nihon Iyakuhin-syu* 1997, October, p. 92, published by *Yakugyo Jiho-sha*).
The effective dose of amorolfine in the antifungal agent of the present invention is 0.1 to 1 % by mass, preferably 0.1 to 0.5 % by mass and more preferably 0.2 to 0.4 % by mass.
In this specification, butenafine, which is a benzylamine antifungal agent, also includes its salts. Butenafine hydrochloride is particularly preferred.
The structural formula of butenafine hydrochloride (N-4-tert-butylbenzyl-N-methyl-1-naphthalenemethylamine hydrochloride ($C_{23}H_{27}N \cdot HCl$), molecular weight: 353.93) is as follows:

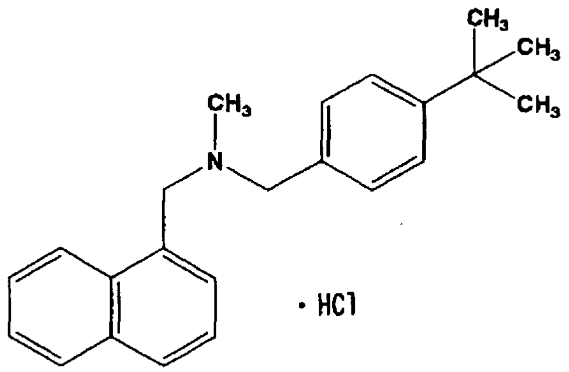

Butenafine is characterized in its strong antifungal activity particularly against dermatophytes such as Trichophyton, Microsporum and Epidermophyton, as well as Malassezia. In Japan, butenafine is an antifungal agent most commonly used as a medicine at present, and it is easily available on the market.
The MIC of butenafine hydrochloride ranged from 0.006 to 0.025 µg/mL against Trichophyton mentagrophytes, and from 0.0015 to 0.025 µg/mL for Trichophyton rubrum (Tetsuya Maeda, et al., Journal of the Pharmaceutical Society of Japan 111, 126-137 (1991) and Mamoru Yokoo, et al., *Nishi-Nihon Hifu-ka* 53, 144-151 (1991)). Butenafine hydrochloride has a very strong antifungal effect on Trichophyton but it is ineffective on Candida.
As for the mechanism of the function of butenafine hydrochloride, it inhibits the synthesis of ergosterol but the site on which it is effective is based on the inhibition of squalene epoxidation reaction unlike that of imidazoles and butenafine hydrochloride has a fungicidal effect.
The toxicity of butenafine hydrochloride is as follows:

$LD_{50}$ (mg/kg) ICR male and female mice: percutaneous > 800, subcutaneous > 200, intravenous injection > 140, oral > 5000;
Wistar rats: percutaneous > male and female 1000, subcutaneous > male and female 150, intravenous injection > male 100, ≧female 100, oral > male and female 4000;
Beagle dogs, male: percutaneous > 100, oral > 5000.

No toxicity was found on the reproduction or development, and no antigenicity or mutagenicity was found. As for the localized irritation, the irritation of the skin on the back and eye mucous membrane of rabbits by the cream or liquid was weak, and no increase in the irritation was observed even in butenafine hydrochloride deteriorated under severe conditions (see *Iryoyaku Nihon Iyakuhin-syu* 1997, October, p. 1262, published by *Yakugyo Jiho-sha*).
The effective dose of butenafine in the antifungal agent of the present invention is 0.1 to 2 % by mass, preferably 0.1 to 1 % by mass and more preferably 0.3 to 1 % by mass.
The mass ratio of amorolfine to butenafine is 100:1 to 1:10, preferably 20:1 to 1:5 and more preferably 5:1 to 1:5.
In this specification, terbinafine, which is an arylamine antifungal agent, also includes its salts. Terbinafine hydrochloride is particularly preferred.
The structural formula of terbinafine hydrochloride ((E)-N-(6,6-dimethyl-2-hepten-4-ynyl)-N-methyl-1-naph-

thalenemethylamine hydrochloride ($C_{21}H_{25}N \cdot HCl$), molecular weight: 327.90) is as follows:

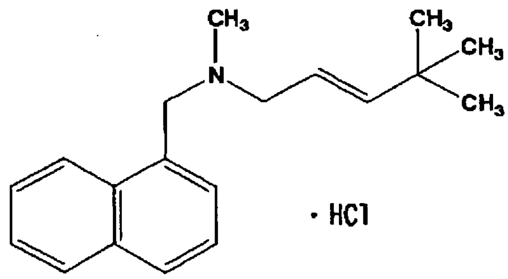

Terbinafine is characterized in its strong antifungal activity particularly against the fungi of dermatophytes such as Trichophyton, Microsporum and Epidermophyton, as well as Malassezia. The antifungal activity of terbinafine against Candida was also confirmed. In Japan, terbinafine is an antifungal agent most commonly used as a medicine at present, and it is easily available on the market.

The MIC of terbinafine hydrochloride ranged from 0.001 to 0.01 μg/mL against Trichophyton mentagrophytes and Trichophyton rubrum. Terbinafine hydrochloride at a low concentration clearly exhibits a fungicidal effect on sterilizing germinated conidium of Trichophyton mentagrophytes (Schuster, I., et al.: "Preclinical characteristics of allylamines"; in Berg, D., et al. eds. Sterol Biosynthesis Inhibitors: Pharmaceutical and Agrochemical Aspects.: Pbl.: Ellis Horwood Ltd. Chichester (UK) pp. 449-470, 1998 and Tamio Hiratani et al. "*Nihon I-Shinkin Gakkai Zassi*" 32 (4), 323, 1991). As for the effect of terbinafine hydrochloride at a concentration of at least 0.098 μg/mL on Candida albicans, it inhibits the conversion from yeast form to mycelial form. Terbinafine hydrochloride at a concentration of at least 1 μg/mL exhibits a fungistatic effect on the propagation of the yeast form (Schaude, M., et al.: Mykosen 30(6), 281, 1987 and Tamio Hiratani, et al.: *Nihon I-Shinkin Gakkai Zassi* 33(1), 9, 1992).

As for the mechanism of the function of terbinafine hydrochloride, it selectively inhibits squalene epoxidase in the cells of fungi. It also causes the accumulation of squalene and reduction of ergosterol content and exhibits the antifungal effect. For dermatophytes, it destroys, even in a low concentration, the cell membrane formation to exhibit the fungicidal effect on the fungi. For Candida albicans, it exhibits a partial growth-inhibiting effect from the low concentration and, at a high concentration, it exhibits antifungal activity by the direct membrance-damaging effect.

The toxicity of terbinafine hydrochloride is as follows:

$LD_{50}$ (mg/kg) mice: intravenous injection = male 410, female 377, oral = male 3570, >female 4000, subcutaneous > male and female 2000;
Rats: intravenous injection = male 220, female 206, oral > male and female 4000, subcutaneous and percutaneous > male and female 2000;
Male and female rabbits: percutaneous > 1500. In the reproduction tests, the suppression on the increase in the body weight of dams of rats and rabbits was recognized when terbinafine hydrochloride was administered in a large amount. However, on the other hand, no influence was recognized on the reproduction and the growth of the embryos. No antigenicity was observed. As for the localized irritation, a weak irritation or a light cumulative irritation of the skin on the back and eye mucous membrane of rabbits was recognized (see *Iryo-yaku Nihon Iyakuhin-syu* 1997, October, pages 959 to 960, published by *Yakugyo Jiho-sha*).

The effective dose of terbinafine is 0.1 to 2 % by mass, preferably 0.1 to 1 % by mass and more preferably 0.3 to 1 % by mass.

The mass ratio of amorolfine to terbinafine is 100:1 to 1:10, preferably 20:1 to 1:5 and more preferably 5 :1 to 1:5.

The antifungal agent for topical use of the present invention can be prepared by an ordinary method (for example, a method specified in Japanese pharmacopoeia, the 14$^{th}$ edition (the preparation of medicines; General Rules for Preparations)). The dosage form of the antifungal agent may be various. The dosage forms for the topical use are, for example, ointment, cream (emulsion ointment), gel, liquid, lotion and aerosol.

The ointments are homogeneous semi-solids having a suitable consistency for topical use and to be applied to the skin. They include oil and fat ointments, emulsion ointments and water-soluble ointments. The emulsion ointments

in the form of a cream are particularly preferred. The cream contains an emulsion base of oil-in-water type such as a hydrophilic ointment, an emulsion base of water-in-oil type such as an absorptive ointment or water-free emulsion base such as hydrophilic Vaseline. The gel is prepared by suspending a hydrate of a water-insoluble medicine in an aqueous liquid. The liquid indicates a liquid preparation for topical use. It includes all the liquid preparations suitable for use as the topical antifungal agent or remedy for athlete's foot according to the present invention. Concretely, the liquids include lotions, suspensions, emulsions, liniments, etc. The lotions are liquid topical preparations to be applied to the skin, which are prepared by dissolving or homogeneously dispersing a medicine in an aqueous liquid. The aerosols are prepared by such a method that a pressure of a liquefied gas or a compressed gas in a vessel ejects a solution, suspension or the like of a medicine at the time of use. The ejection may be in the form of a mist, powder, foam or paste.

In the preparation of the antifungal agent for topical use or remedy for athlete's foot in such a dosage form in the present invention, the amount of amorolfine, butenafine or terbinafine is preferably the above-described effective dose.

The mass ratio of amorolfine to butenafine or amorolfine to terbinafine is preferably as described above.

The antifungal agent for topical use or the remedy for athlete's foot according to the present invention may further contain components usually incorporated into remedies for topical use so far as the effect of the present invention would not be affected. In addition, an excipient, a solubilizer, etc. are usable in the preparation of them. They are, for example, antihistaminic agents (such as diphenhydramine, diphenhydramine hydrochloride, isothipendyl hydrochloride and chlorpheniramine maleate), anti-inflammatory agents (such as glycyrrhizic acid and salts thereof, dipotassium glycyrrhizate, glycyrrhetinic acid and allantoin), disinfectants (such as isopropylmethylphenol, benzethonium chloride, dequalinium chloride and hinokitiol), antipruritic agents (such as crotamiton), refrigerants (such as camphor, menthol and peppermint oil), local anesthetics (such as dibucaine hydrochloride, lidocaine, lidocaine hydrochloride and ethyl aminobenzoate), antioxidants (such as dibutylhydroxytoluene, sodium pyrosulfite, propyl gallate and ascorbic acid), corneous layer solubilizers (such as urea and salicylic acid), gelling agents (such as carboxyvinyl polymers, hydroxyethylcellulose, hydroxypropylcellulose, hydroxypropylmethylcellulose and polyvinylacetal diethylaminoacetate), sequestering agents (such as disodium ethylenediaminetetraacetate (EDTA-2Na)), pH regulators (such as sodium lactate, lactic acid, triethanolamine, diisopropanolamine, citric acid, sodium citrate and sodium hydroxide), oil ingredients (such as liquid paraffin, stearyl alcohol, stearic acid, medium chain fatty acid triglycerides, Vaseline, gelled hydrocarbons and animal and vegetable oils), polyhydric alcohols (such as glycerol, propylene glycol, macrogol, 1,3-butylene glycol, glycerol monostearate and higher alcohols), emulsifying agents (such as glycerol fatty acid esters, sorbitan fatty acid esters, sorbitan monostearate, Spans, polyoxyl stearates, polysorbates (Tweens), polyoxyethylene-hardened castor oil, sodium laurylsulfate and polyoxyethylene sorbitan monostearate), preservatives (such as p-hydroxybenzoic acid salts, benzoic acid salts, benzalkonium chloride and sorbic acid), solvents (such as ethyl alcohol, denatured 99 v/v % ethyl alcohol, and isopropanol), suspending agents (such as gum arabic, sodium alginate, Carmellose sodium, methylcellulose and bentonite), other bases (such as lanolin, paraffin, wax, resins, plastics, glycols and water), propellants for aerosols (such as Freon and substitute Freons (chlorine-free fluorinated hydrocarbons such as HFC-134a and HFC-227) and dimethyl ether (DME).

## Examples

The following Examples and Test Examples will further illustrate the present invention, which by no means limit the scope of the invention.

### Example 1

The following components were mixed together to obtain a liquid.

| Components | Amount (g) |
|---|---|
| Amorolfine hydrochloride | 0.3 |
| Butenafine hydrochloride | 0.4 |
| Crotamiton | 5.0 |
| 1, 3-Butylene glycol | 14.5 |
| Hydroxypropylcellulose | 1.0 |
| Purified water | 10.0 |
| Ethyl alcohol | ad 100 mL |

Example 2

The following components were mixed together to obtain a liquid.

| Components | Amount (g) |
|---|---|
| Amorolfine hydrochloride | 0.2 |
| Butenafine hydrochloride | 0.5 |
| Crotamiton | 5.0 |
| 1,3-Butylene glycol | 24.5 |
| Polyvinylacetal diethylaminoacetate | 2.5 |
| Denatured 99 v/v % ethyl alcohol | ad 100 mL |

Example 3

The following components were mixed together to obtain a liquid.

| Components | Amount (g) |
|---|---|
| Amorolfine hydrochloride | 0.35 |
| Butenafine hydrochloride | 0.35 |
| Crotamiton | 5.0 |
| Diphenhydramine | 0.25 |
| Lidocaine | 1.0 |
| 3-Butylene glycol | 20.0 |
| Hydroxypropylmethylcellulose | 0.5 |
| Purified water | 5.0 |
| Ethyl alcohol | ad 100 mL |

Example 4

The following components were mixed together to obtain a cream.

| Components | Amount (g) |
|---|---|
| Amorolfine hydrochloride | 0.3 |
| Terbinafine hydrochloride | 0.4 |
| Lidocaine | 2.0 |
| Polyoxyethylene sorbitan monostearate | 3.0 |
| Sorbitan monostearate | 1.0 |
| 1,3-Butylene glycol | 10.0 |
| Medium chain triglyceride | 10.0 |
| Stearyl alcohol | 5.0 |
| Glycerol monostearate | 2.5 |
| EDTA-2Na | 0.1 |
| Purified water | ad 100 mL |

Example 5

The following components were mixed together to obtain a gel cream.

| Components | Amount (g) |
|---|---|
| Amorolfine hydrochloride | 0.2 |
| Terbinafine hydrochloride | 0.5 |
| Lidocaine | 2.0 |

(continued)

| Components | Amount (g) |
|---|---|
| Polyoxyethylene sorbitan monostearate | 1.0 |
| Propylene glycol | 10.0 |
| Medium chain triglyceride | 5.0 |
| Stearyl alcohol | 1.0 |
| Carboxyvinyl polymer | 1.0 |
| Diisopropanolamine | 1.0 |
| EDTA-2Na | 0.1 |
| Purified water | ad 100 mL |

Example 6

The following components were mixed together to obtain an aerosol.

| Components | Amount (g) |
|---|---|
| Crude liquid: amorolfine hydrochloride | 0.35 |
| Terbinafine hydrochloride | 0.35 |
| Dipotassium glycyrrhizate | 0.25 |
| Ethanol | 35.0 |
| Purified water | 50 mL |
| Propellant: DME (dimethyl ether) | 50 mL |

(Preparation method)

The crude liquid prepared by dissolving the active ingredients in a base composed of ethanol and purified water was placed in a vessel. Then, a valve was attached thereto. The vessel was filled with the propellant to prepare the aerosol.

Test Example 1

(Specimens)

Specimen 1: Tolnaftate
Specimen 2: Butenafine hydrochloride
Specimen 3: Terbinafine hydrochloride
Specimen 4: Lanoconazole
Specimen 5: Ciclopyrox olamine

Amorolfine hydrochloride and each specimen (hereinafter referred to as "medicine") at a mass ratio of 1:1 were dissolved in dimethyl sulfoxide (DMSO). Two-fold dilution series were prepared with DMSO. In the determination of the antifungal activity, the medicine and the medium were mixed together at a ratio of 1:99.

(Test method)

Sabouraud agar medium *(Eiken)* was used as a susceptibility determination medium. A microbe suspension containing $10^6$ spores/mL of Candida albicans or about $10^6$ conidia/mL of ringworm fungus was prepared. 5 µl of the suspension was inoculated on the Sabouraud agar medium containing the medicine with a micro-planter (a product of Sakuma Seisakusho). Candida albicans was cultured for 2 days and the tinea was cultured for 7 days. To determine the antifungal activity, FIC index (Fractional Inhibitory Concentration Index) was calculated from the lowest drug concentration (MIC: minimum inhibitory concentration, µg/mL) that completely inhibited growth when the culture was completed.

Formula: FIC index = $a/a_0 + b/b_0$

wherein:

a: MIC of amorolfine hydrochloride used in combination with the specimen
$a_0$: MIC of amorolfine hydrochloride used alone
b: MIC of specimen used in a combination of with amorolfine hydrochoride
$b_0$: MIC of the specimen used alone

The effect obtained by the combination was judged on the basis of the following standards (FIC index):
>2: antagonism
2 ≧ and 1<: additive action
1 ≧ : potentiation

(Results)

Table 1

| FIC index obtained by the combination of amorolfine hydrochloride with another medicine | | | |
|---|---|---|---|
| Medicine | Trichophyton mentagrophytes | Trichophyton rubrum | Candida albicans |
| Tolnaftate | 1.25 | 0.35 | 0.38 |
| Butenafine hydrochloride | 0.49 | 0.38 | 0.38 |
| Terbinafine hydrochloride | 0.52 | 0.75 | 0.27 |
| Lanoconazole | 1.00 | 1.25 | 0.53 |
| Ciclopyrox olamine | 1.0 | 1.0 | 0.38 |

It is clear from Table 1 that, when amorolfine hydrochloride was used in combination with butenafine hydrochloride or terbinafine hydrochloride at a mass ratio of 1:1, the FIC index for all of the above-mentioned three species was lower than 1 to indicate a remarkable potentiation effect.
In this connection, Tolnaftate is a thiocarbamic acid antifungal agent; lanoconazole is an imidazole antifungal agent; and Ciclopyrox olamine is a pyridone antifungal agent.

Test Example 2

(Specimens)

Specimen 1: butenafine hydrochloride
Specimen 2: terbinafine hydrochloride

(1) Amorolfine hydrochloride and each specimen (hereinafter referred to as "medicine") at a mass ratio of 100:1 to 1:100 were dissolved in dimethyl sulfoxide (DMSO). Two-fold dilution series were prepared with DMSO. In the determination of the antifungal activity, the medicine and the medium were mixed together at a ratio of 1:99.
(2) Amorolfine hydrochloride and each specimen (hereinafter referred to as "medicine") at a mass ratio of 1:3 to 3:1 were dissolved in dimethyl sulfoxide (DMSO). Two-fold dilution series were prepared with DMSO. In the determination of the antifungal activity, the medicine and the medium were mixed together at a ratio of 1:99.

(Test method)

Sabouraud agar medium *(Eiken)* was used as a susceptibility determination medium. A microbe suspension containing $10^6$ spores/mL of Candida albicans or about $10^6$ conidia/mL of each of two kinds of ringworm fungus was

prepared. 5 µl of the suspension was inoculated on the Sabouraud agar medium with a micro-planter (a product of Sakuma Seisakusho). Candida albicans was cultured for 2 days and the Tinea fungus was cultured for 7 days. To determine the antifungal activity, the FIC index was calculated from the lowest drug concentration (MIC: minimum inhibitory concentration, µg/mL) that completely inhibited growth when the culture was completed.

(Results)

In the process (1) above, when the mass ratio of amorolfine hydrochloride to butenafine hydrochloride or to terbinafine hydrochloride was in the range of, for example, 100:1 to 1:10, the FIC index was about 0.5 to 0.9 and a particularly remarkable potentiation effect was confirmed.

The results of process (2) are shown in the following table.

Table 2

| Minimum inhibitory (MIC) µg/mL | | | | | | | |
|---|---|---|---|---|---|---|---|
| | A | B | A+B (1:3) | A+B (1:2) | A+B (1:1) | A+B (2:1) | A+B (3:1) |
| Trichophyton mentagrophytes | 0.031 | 0.016 | 0.011 | 0.011 | 0.011 | 0.016 | 0.016 |
| Trichophyton rubrum | 0.016 | 0.004 | 0.002 | 0.0028 | 0.004 | 0.0057 | 0.008 |
| Candida albicans | 1.0 | >8.0 | 1.4 | 1.0 | 0.71 | 0.71 | 0.71 |
| A: amorolfine hydrochloride<br>B: butenafine hydrochloride<br>( ): Mass ratio of medicines | | | | | | | |

Two strains of each species were used. The concentration is represented in terms of geometric mean MIC (µg/mL).

Table 3

| FIC index | | | | | |
|---|---|---|---|---|---|
| | A+B (1:3) | A+B (1:2) | A+B (1:1) | A+B (2:1) | A+B (3:1) |
| Trichophyton mentagrophytes | 0.60 | 0.58 | 0.54 | 0.68 | 0.64 |
| Trichophyton rubrum | 0.41 | 0.53 | 0.63 | 0.71 | 0.88 |
| Candida albicans | 0.42 | 0.37 | 0.38 | 0.49 | 0.54 |
| A: amorolfine hydrochloride<br>B: butenafine hydrochloride<br>( ): Mass ratio of medicines | | | | | |

FIC index for Candida albicans was calculated on the basis of MIC of butenafine hydrochloride to be 16.0 µg/mL.

Table 4

| Minimum inhibitory concentration (MIC) µg/mL | | | | | | | |
|---|---|---|---|---|---|---|---|
| | A | T | A+T (1:3) | A+T (1:2) | A+T (1:1) | A+T (2:1) | A+T (3:1) |
| Trichophyton mentagrophytes | 0.031 | 0.011 | 0.008 | 0.008 | 0.008 | 0.008 | 0.016 |
| Trichophyton rubrum | 0.016 | 0.004 | 0.004 | 0.004 | 0.004 | 0.004 | 0.008 |
| Candida albicans | 1.0 | >8.0 | 1.0 | 0.71 | 0.71 | 0.5 | 0.5 |
| A: amorolfine hydrochloride<br>T: terbinafine hydrochloride<br>( ): Mass ratio of medicines | | | | | | | |

Two strains of each species were used. The concentration is represented in terms of geometric mean MIC (µg/mL).

Table 5

| FIC index | | | | | |
|---|---|---|---|---|---|
| | A+T (1:3) | A+T (1:2) | A+T (1:1) | A+T (2:1) | A+T (3:1) |
| Trichophyton mentagrophytes | 0.61 | 0.57 | 0.49 | 0.41 | 0.75 |
| Trichophyton rubrum | 0.81 | 0.75 | 0.63 | 0.50 | 0.88 |
| Candida albicans | 0.32 | 0.27 | 0.38 | 0.34 | 0.38 |
| A: amorolfine hydrochloride<br>B: terbinafine hydrochloride<br>( ): Mass ratio of medicines | | | | | |

FIC index for Candida albicans was calculated on the basis of MIC of terbinafine hydrochloride to be 16.0 μg/mL.

It is clear from Tables 3 and 5 that, when amorolfine hydrochloride was used in combination with butenafine hydrochloride or with terbinafine hydrochloride at a mass ratio of 1:3 to 3:1, the FIC index for all of the three species was lower than 1 to indicate a remarkable potentiation effect.

It should be noted that the remarkable potentiation effect was recognized for the fungi of not only the genus Trichophyton but also of the genus Candida. Tables 2 and 4 indicate that, when each of butenafine hydrochloride and terbinafine hydrochloride was separately applied to Candida albicans, the result was MIC>8.0 μg/mL but, when each of them was used in combination with amorolfine hydrochloride, the MIC was lower than that (1.0 μg/mL) of amorolfine hydrochloride for Candida albicans. In Tables 3 and 5, the FIC index was 0.32 to 0.54 and a very high potentiation effect was obtained.

<u>Test Example 3</u>

(Specimens)

Specimen 1: butenafine hydrochloride
Specimen 2: terbinafine hydrochloride

Amorolfine hydrochloride and each specimen (hereinafter referred to as "medicine") at a mass ratio of 1:2 were dissolved in polyethylene glycol 400 to obtain a final concentration of 0.75 % mass ratio. Each of amorolfine hydrochloride and the medicines was separately dissolved in polyethylene glycol 400 to obtain solutions having the final concentration of 0.75 mass %.

(Test method)

[Animal samples]

Male Hartley guinea pigs (Japan SLC Co., Ltd.; 6 weeks) were used. In the course of the test, they were kept separately from one another in an isolator (Negative rack, *CLEA JAPAN, Inc.*). They could freely have a solid food (G standard, *Nihon Nosan Kogyo K.K.*) and water.

[Preparation of conidial suspension for inoculation]

Trichophyton mentagrophytes TIMM 1189 was cultured on slant Sabouraud glucose agar medium at 27°C for 14 days. Then, sterilized saline containing 0.05 % of Tween 80 was added thereto to obtain conidia. The resultant suspension was filtered through a cell strainer (FALCON Co., Ltd.) to remove large mycelian masses. The number of conidia was counted with hemocytometer. The conidia were diluted to $4\times10^7$/mL with sterilized saline containing 0.05 % of Tween 80 to obtain the conidial suspension for inoculation.

[Method for infection]

The back of each guinea pig was shaved. Then, a circular inoculation part having a diameter of 2 cm was positioned on both sides of the spinal column. A gummed tape was applied thereto and then peeled. These steps was repeated three times, whereby the hair was pulled out of the skin in both parts and, at the same time, the top of the corneous layer of the skin was peeled off. The remaining hair was completely removed with tweezers. 0.05 mL of the conidial suspension for inoculation was applied to the hair-free parts.

[Treatment test method]

Each test group was composed of 5 guinea pigs. The application of 0.1 mL of the medicinal solution prepared as described above once a day was started 3 days after the inoculation. The period of the application was 5 days. In addition to the treatment groups, a control group of the infected guinea pigs, which were not treated, was provided.

[Judgment of pharmacological effect by culture test]

On the $5^{th}$ day after the completion of the application of the medicine, pieces of the local dermal tissue were subjected to the culture test to judge the pharmacological effect thereof. The culture test was conducted as follows: the guinea pig was sacrificed under anesthesia with ether and then the skin was taken from the whole inoculation parts. Thus, ten pieces of the skin in total were obtained from the inoculated parts (5 guinea pigs × 2 pieces of the skin from the inoculated parts). Each piece of the skin from the inoculated part was further cut into 10 pieces to obtain 100 small pieces of the skin in total for each experiment group. Each of the small pieces was placed on Sabouraud glucose agar plate containing 500 µg/mL of cycloheximide, 50 µg/mL of chloramphenicol and 50 µg/mL of sisomycin and then cultured at 27°C. The small pieces on which Trichophyton mentagrophytes grew to form colonies were taken as positive pieces.

(i) Ratio of positive loci:
When at least one of the 10 small pieces taken from one inoculated locus of the skin was the positive after the culture, the inoculated locus was judged to be positive. The ratio of positive loci after culture was calculated from the number of the cultured positive pieces in the total 10 inoculated loci in each experiment group.
(ii) Average intensity of infection

The number of the cultured positive small pieces in the inoculated portion was scored as will be described below to determine the infection. When the number of the positive small skin pieces in the 10 pieces taken from the skin in one inoculated part was 10, 9, 8, 7, 6, 5, 4, 3, 2, 1 or 0, the results were represented as +10, +9, +8, +7, +6, +5, +4, +3, +2, +1 or 0, respectively. Because the number of the inoculated portions in each experiment group was 10, the total of the scores of the 10 inoculated portions was divided by 10 to calculate the average intensity of infection.

[Statistical analysis]

The rate of positive loci after the culture was analyzed by Fisher's exact probability test, and the intensity of infection was analyzed by Tukey's nonparametric multiple-comparison test. The significant level in both methods was 5 %

(Results)

The results of (i) the rate of positive loci and (ii) average intensity of infection are shown in Figs. 1 and 2, respectively. In the control group of the infected guinea pigs that were not treated, the rate of positive pieces after the culture was as high as 100 % and the average intensity of infection was also as high as +10.0. Thus, the infection was mycologically confirmed.

In Fig. 1, the rate of positive loci was 100 % in all of the 3 groups in which a medicine was used alone (amorolfine hydrochloride alone, butenafine hydrochloride alone or terbinafine hydrochloride alone). In other words, when such a medicine was used alone, the fungi were detected in all of the 10 inoculated portions. On the other hand, in the group to which a combination of two kinds of medicines (combination of amorolfine hydrochloride with butenafine hydrochloride or a combination of amorolfine hydrochloride with terbinafine hydrochloride) was administered, the rates of the positive loci were 40 % and 0 %, respectively.

In Fig. 2, the average intensity of infection was 4 to 5 in the groups in which only one of the 3 medicines was used alone, and the average intensity of infection was 0.4 and 0 in the groups in which a combination of two kinds of

medicines was used. Namely, when the medicines are used in combination with each other, the resultant therapeutic effect is significantly higher than that in the groups in which the medicines were used separately. In particular, when a solution of a combination of amorolfine hydrochloride with terbinafine hydrochloride was used, surprisingly, the fungi were not found in any of the inoculated portions or in any of the small skin pieces to indicate a remarkably high treatment effect. It was confirmed that the antifungal agent comprising a combination of amorolfine hydrochloride with butenafine hydrochloride or a combination of amorolfine hydrochloride with terbinafine hydrochloride exhibits a remarkable potentiation also *in vivo.*

As described above in detail, the antifungal agent of the present invention comprising the combination of amorolfine with butenafine or terbinafine exhibits a remarkable potentiation effect. The present invention can provide the excellent antifungal agent and remedy for athlete's foot that exhibit an antifungal activity on the microorganisms of both ringworm fungi and Candida, even when they are used at a low concentration.

## Claims

**1.** An antifungal agent for topical use, comprising amorolfine and butenafine.

**2.** The antifungal agent of claim 1, wherein the mass ratio of amorolfine to butenafine is in the range of 100:1 to 1:10.

**3.** An antifungal agent for topical use, comprising amorolfine and terbinafine.

**4.** The antifungal agent of claim 3, wherein the mass ratio of amorolfine to terbinafine is in the range of 100:1 to 1:10.

**5.** A remedy for athlete's foot, comprising amorolfine and butenafine.

**6.** The remedy for athlete's foot of claim 5, wherein the mass ratio of amorolfine to butenafine is in the range of 100: 1 to 1:10.

**7.** A remedy for athlete's foot, comprising amorolfine and terbinafine.

**8.** The remedy for athlete's foot of claim 7, wherein the mass ratio of amorolfine to terbinafine is in the range of 100: 1 to 1:10.

FIG.1

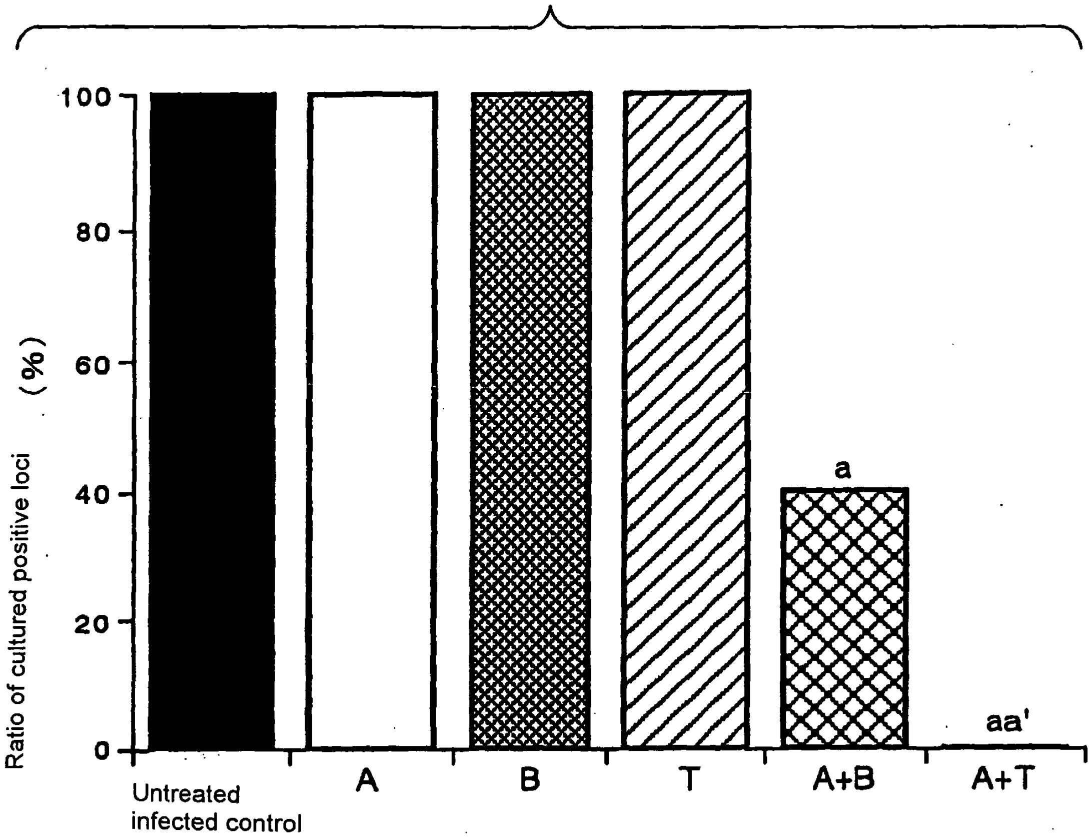


(n=10)

A: Amorolfine hydrochloride, B: Butenafine hydrochloride, T: Terbinafine hydrochloride

a : Significant difference from the untreated group

a' : Significant difference from A+B treated group

FIG.2

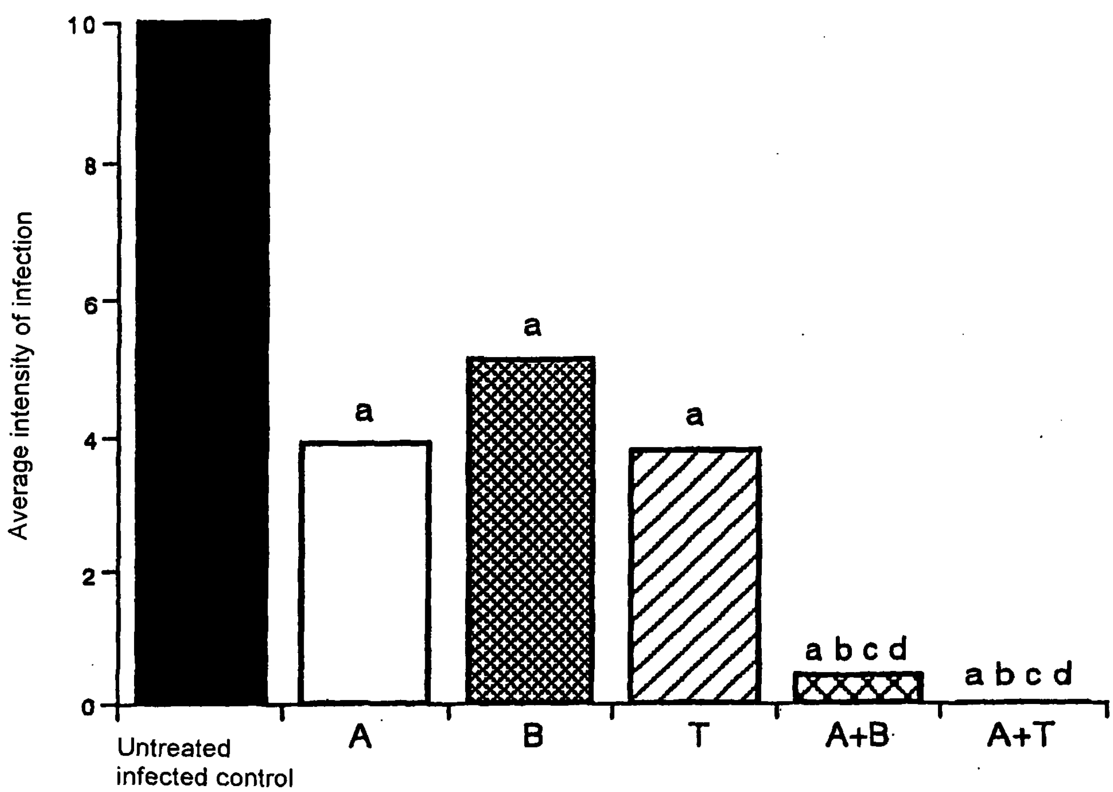


(n=10)

A: Amorolfine hydrochloride, B: Butenafine hydrochloride, T: Terbinafine hydrochloride

a: Significant difference from the untreated group

b: Significant difference from the A treated group

c: Significant difference from the B treated group

d: Significant difference from the T treated group

## INTERNATIONAL SEARCH REPORT

International application No.
PCT/JP03/08221

A. CLASSIFICATION OF SUBJECT MATTER
Int.Cl$^7$ A61K31/137, 31/5375, A61P31/10

According to International Patent Classification (IPC) or to both national classification and IPC

B. FIELDS SEARCHED
Minimum documentation searched (classification system followed by classification symbols)
Int.Cl$^7$ A61K31/137, 31/5375

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | Baran, R.; Feuilhade, M.; Datry, A.; Goettmann, S.; Pietrini, P.; Viguie, C.; Badillet, G.; Larnier, C.; Czernielewski, J., A randomized trial of amorolfine 5% solution nail lacquer combined with oral terbinafine compared with terbinafine a lone in the treatment of dermatophytic toenail onychomycoses affecting the matrix region, British Journal of Dermatology (2000), 142(6), 1177-1183 | 3,4,7,8 |
| Y<br>A | JP 8-20527 A (Toko Yakuhin Kogyo Kabushiki Kaisha),<br>23 January, 1996 (23.01.96),<br>Full text<br>& EP 715856 A | 3,4,7,8<br>1,2,5,6 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July, 2003 (28.07.03) | 12 August, 2003 (12.08.03) |
| Name and mailing address of the ISA/<br>Japanese Patent Office | Authorized officer |
| Facsimile No. | Telephone No. |

Form PCT/ISA/210 (second sheet) (July 1998)